# EUROPEAN PATENT SPECIFICATION

(11) **EP 0 850 427 B1**
(45) Date of publication and mention of the grant of the patent: **27.03.2002**
(21) Application number: 96926107.2
(22) Date of filing: 22.07.1996
(51) Int. Cl.: C07D 498/10, G03C 1/685

(54) **PHOTOCHROMIC SPIROXAZINES, COMPOSITIONS AND ARTICLES CONTAINING THEM**
PHOTOCHROME SPIROXAZINE, ZUBEREITUNGEN UND DIESE ENTHALTENDE GEGENSTÄNDE
SPIROXAZINES PHOTOCHROMIQUES, COMPOSITIONS ET ARTICLES CONTENANT CES COMPOSES

(30) Priority: 30.08.1995 FR 9510221; 16.01.1996 US 9855
(43) Date of publication of application: 01.07.1998
(73) Proprietor: CORNING INCORPORATED, Corning, N.Y. 14831 (US)
(72) Inventor: CHAN, You-Ping, F-69008 Lyon (FR)
(74) Representative: Le Roux, Martine
(86) International application number: US9612083
(87) International publication number: WO9708573

(56) References cited:
- DE-A- 1 927 849
- US-A- 4 913 544
- US-A- 5 114 621
- US-A- 5 139 707
- US-A- 5 446 151
- US-A- 5 529 725

## Description

The present invention concerns novel compounds of the spiroxazine type presenting, in particular, photochromic properties. It also concerns the photochromic compositions and ophthalmic articles (e.g., lenses) which contain these spiroxazines.

The photochromic compounds are capable of changing color due to the influence of a poly- or monochromatic light (e.g., UV) and they are capable of recovering their initial color when the irradiation with light stops, or due to the influence of a poly- or monochromatic light which is different from the first light, or due to the influence of temperature and/or of a poly- or monochromatic light which is different from the first one.

These photochromic compounds are applied in various fields, for example, for the manufacture of ophthalmic lenses, contact lenses, some sunshades, filters, optics for cameras or photographic apparatus or other optical and observation devices, glass partitions, decorative objects, display elements or for the storage of information by optical inscription (coding).

In the field of ophthalmic optics, and in particular in the field of eyeglasses, a photochromic lens, comprising one or more photochromic compounds, must present:
- a high transmission in darkness or in the absence of sunlight,
- a low transmission (high colorability) when exposed to irradiation by sunlight,
- an appropriate kinetics of coloration and decoloration,
- a tint which is acceptable to the consumer (gray or chestnut brown, preferably), with, preferably, maintenance of the selected tint during the course of the coloration and the decoloration of the lens,
- a maintenance of the performances of the characteristics in a temperature range of 0-40°C,
- a significant durability, because the objectives intended are sophisticated, and therefore expensive, corrective lenses.

These lens characteristics are in fact determined by the active photochromic compounds which, in addition, must be perfectly compatible with the organic or mineral support which constitutes the lens.

Moreover, it should be noted that the obtention of a gray or chestnut brown tint may require the use of at least two photochromes of different colors, that is, having distinct maximum absorption wavelengths in the visible range (λₘₐₓ). This association thus imposes still other requirements on photochromic compounds. In particular, the kinetics of coloration and decoloration of the two or more associated active photochromic compounds must be essentially identical. The same applies to their stability over time and, also, to their compatibility with a plastic or mineral support.

Among the numerous photochromic compounds described in the prior art, one can cite the indolinospironaphtoxazines described in U.S. Patent Nos. 3,578,602; 3,562,172; 4,215,010; 5,114,621; 5,139,707; European Patent Nos. 0,171,909; 0,313,941; French Patent No. 2,647,789 and European Patent No. 0,600,669:

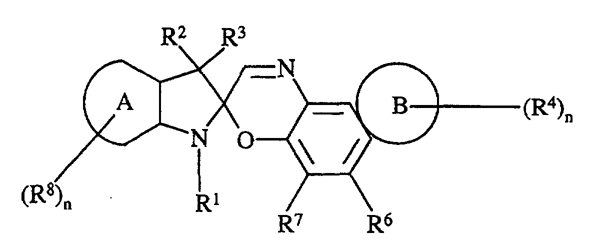

The group R¹ of these molecules represent straight or branched alkyls, alkylaryls or alicyclics. These compounds are considered to meet the specifications defined above.

In fact, while these compounds have indeed one or more of the basic properties sought, such as a high transmission in darkness, a high colorability when exposed to sun radiation, absorption in the blue or violet (570-630 nm), a rapid kinetics of coloration and decoloration, all the compounds described to this day do not have the complete combination of the wanted properties which is required for the production of satisfactory articles which can be manufactured industrially.

Whereas the prior art teach how to modify the absorption band by the addition of substituents to the different positions of the rings and, also, teach how to modify the kinetics of decoloration, in contrast, it does not teach how to increase the colorability of these molecules without increasing the residual coloration in the inactivated state and, above all, on how to make them photochemically stable so as to allow their use on an industrial scale. Indeed, without a high stability, these expensive molecules, introduced into a sophisticated lens, cannot be used.

It is the merit of the applicant to have found, unexpectedly, that the presence of polycyclic groups, preferably bicycloalkyl groups, allowed a solution of the problem of the stability, the residual coloration and the colorability, which are essential for the above-mentioned applications.

The originality of the invention resides in the surprising effect of the polycyclic groups, which increases the colorability of the spiroxazines without increasing their residual coloration, while ensuring excellent photostability.

Thus, the present invention concerns a compound, in particular a photochromic compound, having the following general formula (I): 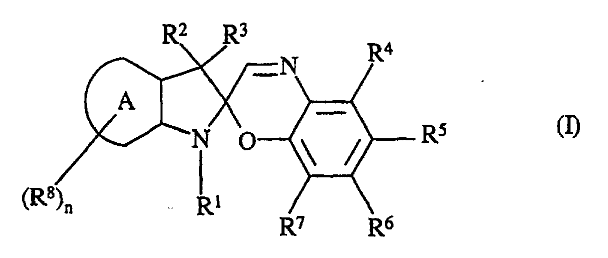 in which:
- R¹ is a polycyclic group formed by at least one alicyclic group which is linked to, bridged or condensed with at least one other aliphatic and/or aromatic ring, where the rings can optionally contain at least one heteroatom and at least one unsaturation,
- R², R³ are identical or different and they represent an alkyl group, straight or branched, of 1-12 carbon atoms, an alkenyl group, an alkynyl group, an aryl group, an alkylaryl group, a cycloalkyl group, R² and R³ can, optionally, combine to form a carbocyclic or heterocyclic group having 5 to 10 atoms,
- R⁴, R⁵, R⁶ and R⁷ are identical or different and they represent:
   ■ hydrogen,
   ■ an alkyl, cycloalkyl, alkenyl, alkynyl, aryl (preferably phenyl, naphthyl mono-, -di- or trisubstituted by electron donor or acceptor groups), heteroaryl, aryloxy or aralkyl, said group being optionally halogenated,
   ■ a halogen, preferably F, Br, Cl,
   ■ OR, SR, -OCOR, or -COOR, with R = H, alkyl and/or cycloalkyl and/or aryl,
   ■ a (poly)ether, a (poly)amide, a (poly)carbonate, a (poly)carbamate, a (poly)urea or a (poly)ester,
   ■ ∗ an amino radical which gives rise, once it is bound in (I), to a primary, secondary or tertiary amine, said amine being alkyl, aryl or aralkyl, mono- or disubstituted depending on its nature,
      ∗ or an aminocyclic radical containing, optionally, one or more heteroatoms,
   ■ or an electron withdrawing group selected, preferably, from the group comprising CF₃, CN, NO₂, SCN,
- where at least two of the radicals R⁴, R⁵, R⁶, R⁷, preferably carried by two adjacent carbon atoms, can combine to form at least one aromatic ring having 5 or 6 members or an aliphatic ring having 5 to 7 members, advantageously 5 or 6 members, said ring(s) comprising, optionally, at least one heteroatom, so as to form at least one heterocyclic ring, the latter being optionally substituted by one or more radicals which may be identical or different, and have the same definition as given above for R⁴ to R⁷,
- A represents a (hetero)aromatic ring (containing, for example, at least one nitrogen atom), which is possibly substituted by one or more radicals R⁸, which may be identical or different and have the same definition as given above for R⁴ to R⁷, except hydrogen,
- n is a whole number and when n ≥ 2, two of the radicals R⁸ can possibly combine to form at least one aromatic or heteroaromatic ring.

Preferably, R¹ represents a bicyclic group. It is particularly preferred for R¹ to represent an asymmetric bicyclic group. The asymmetry can be the result of either the nature of the group or the presence of a substituent(s). Preferred examples of such asymmetric bicyclic groups are substituted or unsubstituted norbornyl groups.

According to the invention it is possible to consider the substitution of at least one of the rings of R¹ by at least one substituent R⁹ which has the same definition as that given above for R⁴ to R⁷.

Specific examples of groups R¹ are the following: 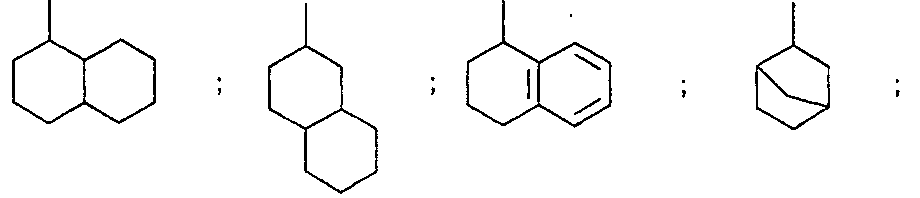 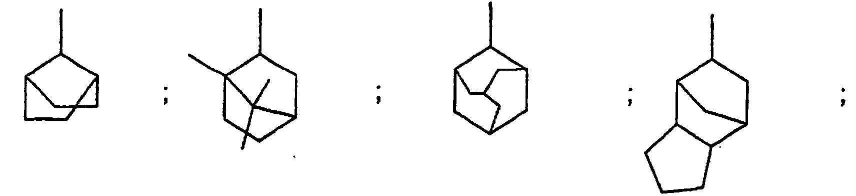 among many others.

Preferred compounds of the invention have the following formula (I'): 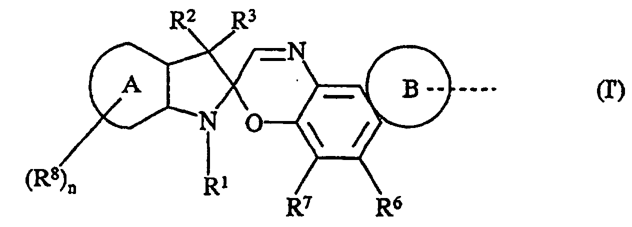 where A, R¹ to R³, R⁶ to R⁸ and n are as defined above, and B is an aromatic or aliphatic ring having 5 to 7 members, optionally comprising a heteroatom, which may or may not be substituted by one or more radicals, which may be identical or different and have the same definition as given for R⁴ to R⁷, except hydrogen

According to a particularly preferred embodiment of the invention, ring A is a phenyl group and R⁴ and R⁵ combine to form an annealed aromatic ring or a bicyclic aromatic ring condensed with the phenyl group which bears them. This corresponds to the following formula (I"): 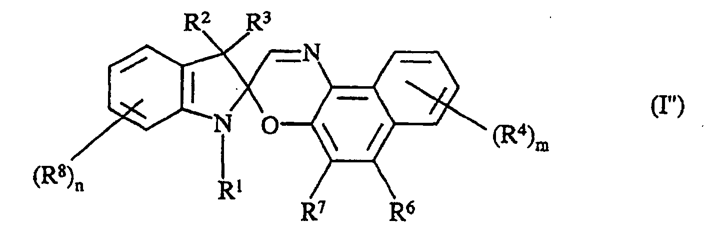 in which R¹ to R⁴ and R⁶ to R⁸ are as defined above, and
n assumes the values 0 to 4, while m assumes the values 1 to 4.

Among the substituents which can be considered for the compounds with formula (I), (I') and (I") according to the invention, groups R⁴ to R⁹ must be considered, which comprise and/or form at least one reactive function for polymerization and/or crosslinking, selected, preferably, from the following list: alkenyl, advantageously vinyl, methacryloyl, acryloyl, acryloxyalkyl, methacryloxyalkyl or epoxy.

Thus, the photochromic compounds according to the invention can be designed as monomers, of different types or not, which can react between themselves or with other comonomers, to form homopolymers and/or copolymers which carry a photochromic functionality and which have the mechanical properties of macromolecules. It follows that one of the objects of the present, invention are formed by these homopolymers or copolymers comprised of the (co)monomers and/or by the crosslinkages, at least in part made up of photochromic compounds (I), (I') and (I") according to the invention.

In the same order of ideas, the above-mentioned compounds (I), (I') and (I") can be considered to be crosslinking agents which have reactive functions capable of allowing the formation of bridges between polymer chains of photochromic nature or not. The crosslinked compounds which can thus be obtained, also constitute another object of the present invention.

In a general manner, in the preceding formulas, the following designations are used, according to the invention:
- "alkyl," referring preferably to a straight or branched hydrocarbon group having from 1 to 12 carbon atoms;
- "alkoxyl," referring to a group of O-alkyl type preferably having from 1 to 10 carbon atoms,
- "aryl," referring to an aromatic hydrocarbon group containing at least 6 carbon atoms,
- "heteroaryl," referring to an aromatic hydrocarbon group comprising at least 5 atoms, of which at least one is a heteroatom,
- "aralkyl," a group comprising at least one alkyl and at least one aryl, as defined above,
- "heteroatom," atoms different from C and H, belonging preferably to the following group: N, O, S and P.

The photochromic compounds which are used particularly preferentially in the context of the invention thus are, as can be concluded from the above, indolinospironaphthoxazines or indolinospirobenzoxazines.

The most advantageous indolinospiroxazines include those having the formula: 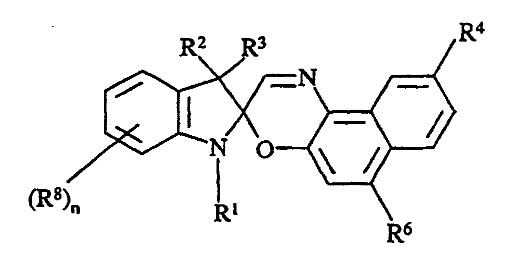 where
N-R¹ = 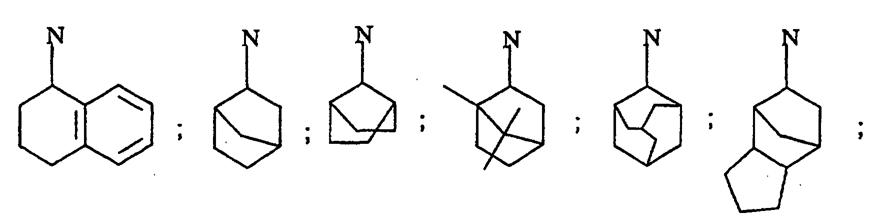
n = 0, 1 or 2 and
R², R³ = C₁-C₅ alkyl,
R⁴ = H, OMe,
R⁶ = H, OMe or amino,
R⁸ = Me, OMe or CF₃.

It is the merit of the applicant to have disclosed these compounds, because they present particularly advantageous photochromic properties. More specifically, they have a high colorability, particularly in the blue region. They are thus well suited to combination, observing compatibility and complementarity requirements, with photochromes which absorb in the yellow, orange, red and violet, so as to obtain a broad coverage of the visible absorbance spectrum and thus coloration tints which are chestnut brown or dark gray.

The sensitivity, as well as the height and the area of their λₘₐₓ peaks in the visible, attain satisfactory values.

These compounds are also perfectly stable and compatible with support matrixes made of organic polymer or of mineral material, both in a form included in the matrix and in the form of a coating.

In solution or in a polymer matrix, the compounds according to the invention are colorless or slightly colored in the initial state and they rapidly develop an intense coloration under UV light (365 nm) or a light source of the solar type. Finally, they quickly recover their initial color when the irradiation stops.

The compounds of the invention can be obtained by the condensation of an indoline derivative substituted with a polycyclic R¹ group and an aromatic nitroso alcohol derivative such as those described, for example, in U.S. Patent Nos. 3,578,602; 4,634,767; 4,913,544 and European Patent No. 600,669. This reaction can take place in solvents such as ethanol, toluene or dichloroethane.

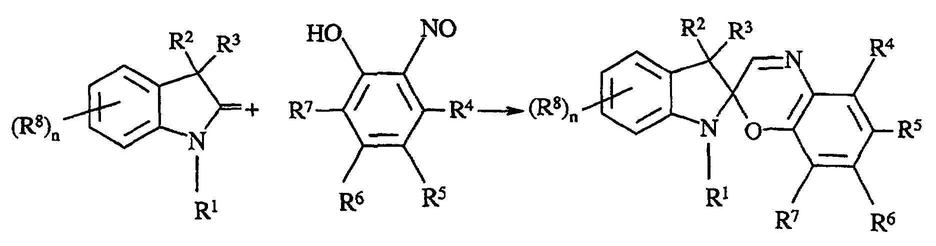

The indoline derivatives are obtained by methods which are adapted from the literature.

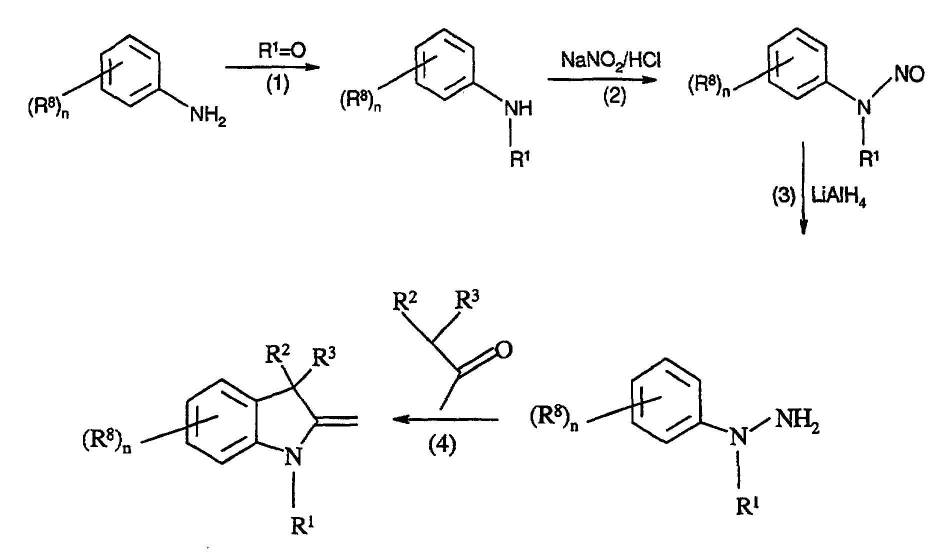

Step 1 is carried out according to a procedure described in Katritzky et al., Tetrahedron 47:2683, 1991. The nitrosation of the amine (step 2) is carried out by a reaction with sodium nitrite-hydrochloric acid and the reduction of the nitroso derivative (step 3) is carried out by the reaction of LiAlH₄ in THF (Fridman et al., Russian Chemical Reviews 40(1):34.197). The last step of the synthesis (4) is carried out by reacting hydrazine with the appropriate ketone in an acidic medium, for example, hydrochloric acid/ethanol or acetic acid (for a general review of this reaction one can consult Robinson "Fischer indole synthesis," Wiley-Interscience, 1982).

In the case of applications of compounds according to the present invention, it should be noted that they can be used as a photochromic material which is dispersed in the superficial part or in the composition of a polymer or mineral matrix. They can also be used in solution.

A photochromic solution can be obtained by dissolving the compound in an organic solvent such as toluene, dichloromethane, tetrahydrofuran or ethanol. The solutions obtained are, in general, colorless and transparent. When exposed to sunlight, they developed a strong coloration and their colorless state returns when they are placed in a zone with less exposure to sunlight or, in other words, when they are no longer exposed to UV radiation. In general, it is sufficient to use a very small concentration of product (on the order of 0.01-5%) to obtain an intense coloration.

The most intense applications are those in which the photochrome is dispersed uniformly within or on the surface of a polymer, copolymer or mixture of polymers. A great variety of methods of implementation can be considered.
Those known to persons skilled in the art include, for example, diffusion in the (co)polymer, from a suspension or solution of the photochrome, in a silicone oil, in an aliphatic or aromatic hydrocarbon, in a glycol, or from another polymer matrix. The diffusion is routinely carried out at a temperature of 50-200°C for a duration of 15 min to several hours, depending on the nature of the polymer matrix.

Another implementation technique consists in mixing the photochrome in a formulation of polymerizable substances, in depositing this mixture on a surface or in a mold and in then conducting the polymerization.

These implementation techniques and others are described in the article by Crano et al. "Spiroxazines and their use in photochromic lenses" published by Applied Photochromic Polymer Systems, Publisher, Blackie and Son Ltd., 1992.

According to a variant of the invention, it is also possible to consider grafting the photochromes onto (co)polymers. Thus, another object of the invention consists of (co)polymers to which at least one of the photochromes described above has been grafted.

Examples of preferred polymer materials for the optical applications of the photochromic compounds according to the invention include the following products:
- alkyl, cycloalkyl, aryl or arylalkyl (mono, di, tri or tetra) polyacrylate or polymethylacrylate, optionally halogenated or comprising at least one ether and/or ester and/or carbonate and/or carbamate and/or thiocarbamate and/or urea and/or amide group,
- polystyrene, polycarbonate (e.g., bisphenol-A polycarbonate, allyl diethylene glycol polycarbonate), polyepoxy, polyurethane, polythiourethane, polysiloxane, polyacrylonitrile, polyamide, aliphatic or aromatic polyester, vinyl polymers, cellulose acetate, cellulose triacetate, cellulose acetate-propionate or polyvinylbutyral,
- copolymers of two or several types of monomer or mixtures of polymers mentioned above, preferably polycarbonate-polyurethane, poly(meth)acrylate-polyurethane, polystyrene-poly(meth)acrylate or polystyrene-polyacrylonitrile, advantageously a mixture of polyester and of polycarbonate or of poly(meth)acrylate.

The quantity of photochrome used depends on the desired degree of darkening. Usually a quantity of 0.001-20 wt% is used.

The photochromic compounds according to the invention can be used alone or in a mixture with other products to form a composition which can be in solid or liquid form, for example, in a solution or in a dispersion, as already indicated above. These compositions, which constitute another object of the invention, can thus comprise the compounds (I), (I') of the invention and other complementary photochromic compounds which allow the obtention of dark colorations, for example, gray or brown, which are desired by the public in applications such as eyeglasses or shades. These complementary photochromic compounds have an λₘₐₓ and an absorbance area surface in the visible such that, after association with the compounds of the invention, an absorbance spectrum is obtained which covers the entire visible spectrum and which imparts the desired tint to the mixture of activated photochromes.

The photochrome(s), which can be combined with the compounds of the invention, is/are those known to a person skilled in the art and described in the literature, for example, chromenes (U.S. Patent Nos. 3,567,605; 5,238,981; World Patent No. 9,422,850; European Patent No. 0,562,915), spiropyrans or naphthospyropans [sic; naphthospiropyrans] (U.S. Patent No. 5,238,981) and spiroxazines (J. C. Crano et al., "Applied Photochromic Polymer Systems," Publisher, Blackie & Son Ltd., 1992, Chapter 2).

These compositions according to the invention can also comprise:
- nonphotochromic dyes which allow an adjustment of the tint,
- and/or one or more stabilizers, such as, for example, an antioxidant,
- and/or one or more anti-UV agents,
- and/or one or more antiradical compounds,
- and/or one or more deactivators of photochemical excitation states.

These additives can allow an improvement of the durability of said compositions.

According to another of its aspects pertaining to the application of the photochromic compounds (I), (I'), the present invention also relates to ophthalmic articles, such as eyeglass or sunshade articles, comprising at least one compound according to the invention and/or at least one (co)polymer formed, at least in part, of recurrent units of type (I), (I') and/or at least one composition comprising the compounds (I), (I') according to the invention, as defined above, and/or at least one matrix, as defined above, made of an organic polymer material or of a mineral material or of a mineral-organic hybrid material incorporating at least one compound of the invention.

In practice, the articles which are more particularly referred to by the present invention are photochromic eyeglass lenses or shades, glare partitions (windows for buildings, for locomotives, automobiles), the optical devices, the decorative articles, the sun protection articles, the storage of data, etc.

The present invention will be understood better in the light of the following examples of photochromic synthesis and validation of compounds (I), (I') and (I"), which it concerns.

### Examples

### Synthesis and properties of photochromic compounds (1)-(8) according to the invention (Examples 1-8)

The formulae of compounds (1)-(8) are given below (see Table I).

### Example 1: Synthesis of compound (1)

### Step 1

### Synthesis of 2-norbornylphenylamine

In a 250 mL flask equipped with a Dean-Stark separator, the following mixture is brought to reflux: 9.3 g of aniline, 12.1 g 2-norbornanone, 13.2 g benzotriazole and 120 mL xylene. After 16 h, the mixture is reduced to dryness, then it is solubilized in 300 mL methanol, and the product is reduced with 6 g NaBH₄ at 50°C for 1 h. The mixture is then poured into 200 mL water and the organic product is extracted with 3 x 100 mL toluene. The organic phase is recovered, dried over magnesium sulfate, and then reduced to dryness. In this manner, 20 g of the desired amine are produced.

### Step 2

### Synthesis of 1-(2-norbornylphenyl)-1-phenylhydrazine

The amine from the preceding step (20 g) is suspended in 100 mL of hydrochloric acid (IN), and then the mixture is maintained at 0°C with stirring. An aqueous solution of NaNO₂ (7 g in 20 mL of water) is then added to the mixture in small portions. The temperature is then allowed to rise to the ambient temperature, and the nitroso derivative is extracted with 3 x 100 mL toluene. After evaporation of the solvent, 26 g of product are recovered. This product is then added slowly and in small portions into tetrahydrofuran (200 mL) containing 7 g LiAlH₄ and, then the mixture is maintained at ambient temperature for 1 h. Subsequently, the mixture is cooled to 0°C, and then the excess hydride is neutralized with an aqueous sodium hydroxide solution. Next, 30 g Na₂SO₄ are added, and the organic phase is recovered by filtration and reduced to dryness. In this manner 21 g of the desired hydrazine are produced.

### Step 3

### Synthesis of the 2-methyleneindoline derivative

In a 100 mL flask, 21 g hydrazine from the preceding step and 8.6 g 3-methyl-2-butanone in 100 mL ethanol containing 2 mL acetic acid at 50°C are reacted. Then 15 mL of concentrated hydrochloric acid are added and brought to reflux for 30 min. The mixture is then neutralized with sodium hydroxide to pH 10 and the indole derivative is extracted with 3 x 100 mL isopropyl ether. After evaporation of the solvent, 17 g of the desired product are prepared.

### Step 4

### Synthesis of spiroxazine (1)

The product from the preceding step (3 g) and 1.5 g 1-nitroso-2-naphthol are dissolved in 50 mL of absolute ethanol, and then the mixture is heated at 60°C for 1 h. The mixture is then cooled to 0°C. After 30 min, the precipitated product is recovered by filtration, and washed with ethanol (20 mL). The solid is then recrystallized in ethanol. 820 mg of the desired product are isolated after filtration. Its structure is confirmed by NMR spectroscopy and the latter reveals, in addition, the existence of two isomers (because of the end- or exo- position of the nitrogen on the norbornyl ring).

### Example 2: Synthesis of compound (2)

The product from step 3 of Example 1 (2.6 g) and 1.9 g 1-nitroso-2,7-dihydroxynaphthalene are dissolved in 130 mL ethanol, and the mixture is brought to reflux for 5 h. The spiroxazine which is hydroxylated in the 9' position is isolated from the reaction mixture by chromatography on a silica column with a toluene/methanol mixture (9/1) as eluant. The methylation of the product is then carried out with dimethyl sulfate in acetone, in the presence of potassium carbonate and at 30°C for 4 h. 1.8 g spiroxazine (2) are isolated after purification by chromatography on a silica column with a toluene/heptane (1/1) mixture as eluent. Its structure is confirmed by NMR spectroscopy.

### Example 3: Synthesis of compound (3)

Compound (3) is synthesized in a similar manner to that of Example 1. In Example 1, 3,4-dimethylaniline is used instead of aniline. Steps 2 and 3 lead to the indoline derivative, which is then condensed with 1-nitroso-2-naphthol to yield spiroxazine 3. It is purified as above by chromatography on a silica column. Its structure is confirmed by NMR spectroscopy. The latter reveals the existence of a mixture of two dimethyl isomers (position 4,5 and 5,6 on the phenyl ring of the indole).

### Example 4: Synthesis of compound (4)

Compound 4 is synthesized in a manner similar to that of Example 2. In step 4, the indoline derivative prepared in Example 3 and 1-nitroso-2,7-dihydroxynaphthalene are used. The intermediate obtained is then methylated with dimethyl sulfate in acetone in the presence of potassium carbonate. The spiroxazine is purified as above by column chromatography on silica. Its structure is confirmed by NMR spectroscopy.

### Example 5: Synthesis of compound (5)

This compound is synthesized in a manner similar to that of Example 1, using as the starting product camphor instead of 2-norbornanone in step 1 of the synthesis.

### Example 6: Synthesis of compound (6)

This compound is synthesized in a manner similar to that of Example 1, using as the starting product 3,4-dimethylaniline and camphor in step 1 of the synthesis.

### Example 7: Synthesis of compound (7)

This compound is synthesized in a manner similar to that of the preceding example with 3,5-dimethylaniline instead of 3,4-dimethylaniline in step 1 of the synthesis.

### Example 8: Synthesis of compound (8)

This compound is synthesized in a manner similar to that of the Example 3 with 3-methyl-2-pentanone instead of 3-methyl-2-butanone in step 3 of the synthesis.

### Applications

### Example 9: Incorporation of compounds (1) through (8) in a polyacrylate

General procedure: 10 mg of each one of compounds (1) through (8) are dissolved in tetraethoxylated bisphenol A dimethyl methacrylate, marketed under the name DIACRYL 121 by the company AKZO and also containing 40 mg 2',2'-azobis(2-methylbutyronitrile). The solution is then degassed, rendered inert with argon and then poured into a glass lens mold having a diameter of 8 cm and a thickness of 2 mm. The mold is then placed in an oven at 70°C for 12 h. After removal from the mold, a transparent and rigid lens is obtained. When exposed to solar radiation, the glass quickly develops an intense blue coloration and it becomes colorless again in darkness. The photochromic characteristics are given in Table I below. For comparison, the characteristics of compounds C1, C2, C3, C4 and C5 of the prior art are also given in Table I below.

### Table I

### Legends:

- λₘₐₓ measured in D121 in a thickness of 2 mm with exposure to a xenon lamp, 60,000 lx, at 22°C,
- T0 = initial transmission (inactivated state) measured at λₘₐₓ,
- TD15 = transmission after 15 min of exposure measured at λₘₐₓ,
- IOD = Induced optical density {log(T0/TD15)},
- R5 = % of recovery of the initial transmission after 5 min of decoloration,
- (Y0/YD15)₀ₕ = initial integrated transmission and transmission after 15 min of exposure, respectively, and before ageing,
- (Y0/YD15)₃₀₀ₕ = initial integrated transmission and transmission after 15 min of exposure, after 300 h of ageing under an exposure to 60,000 lx.

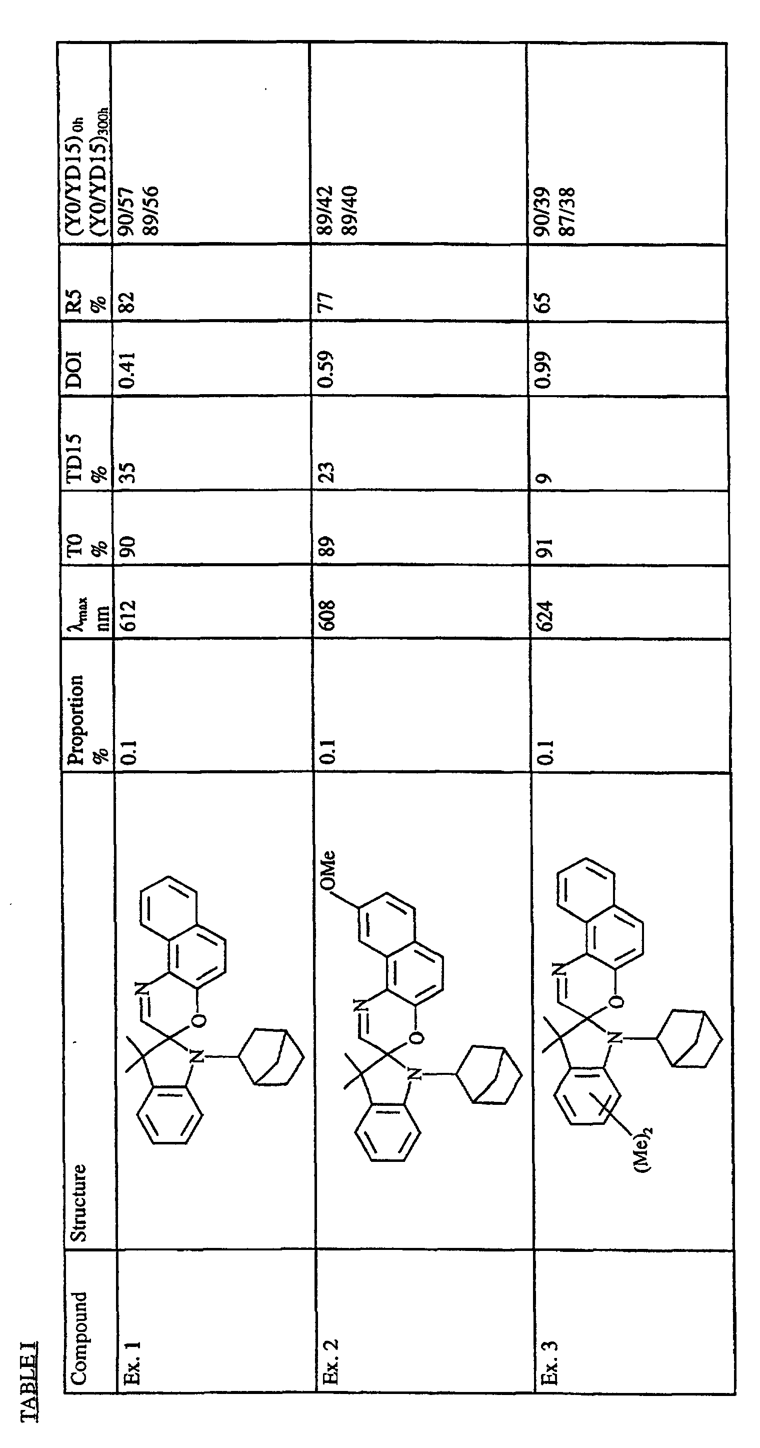

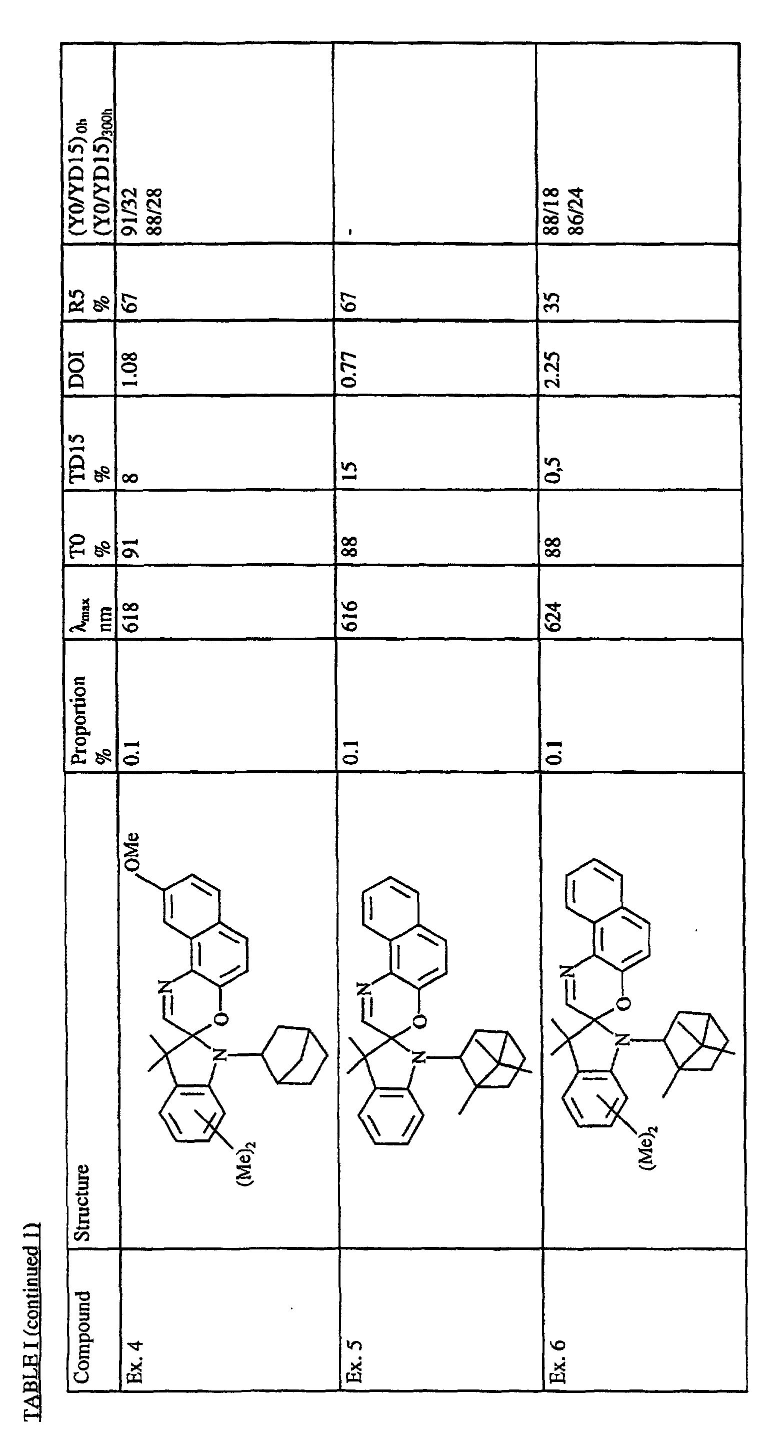

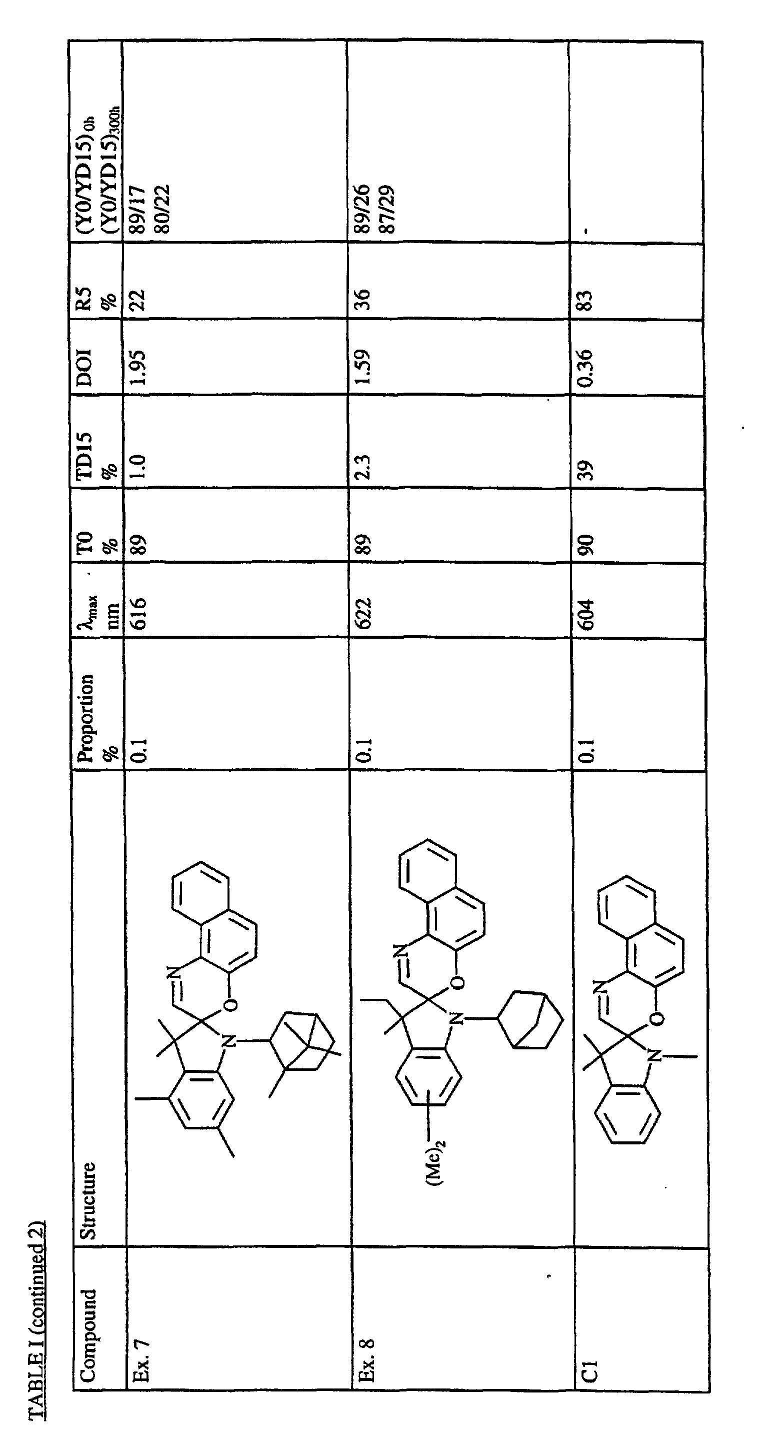

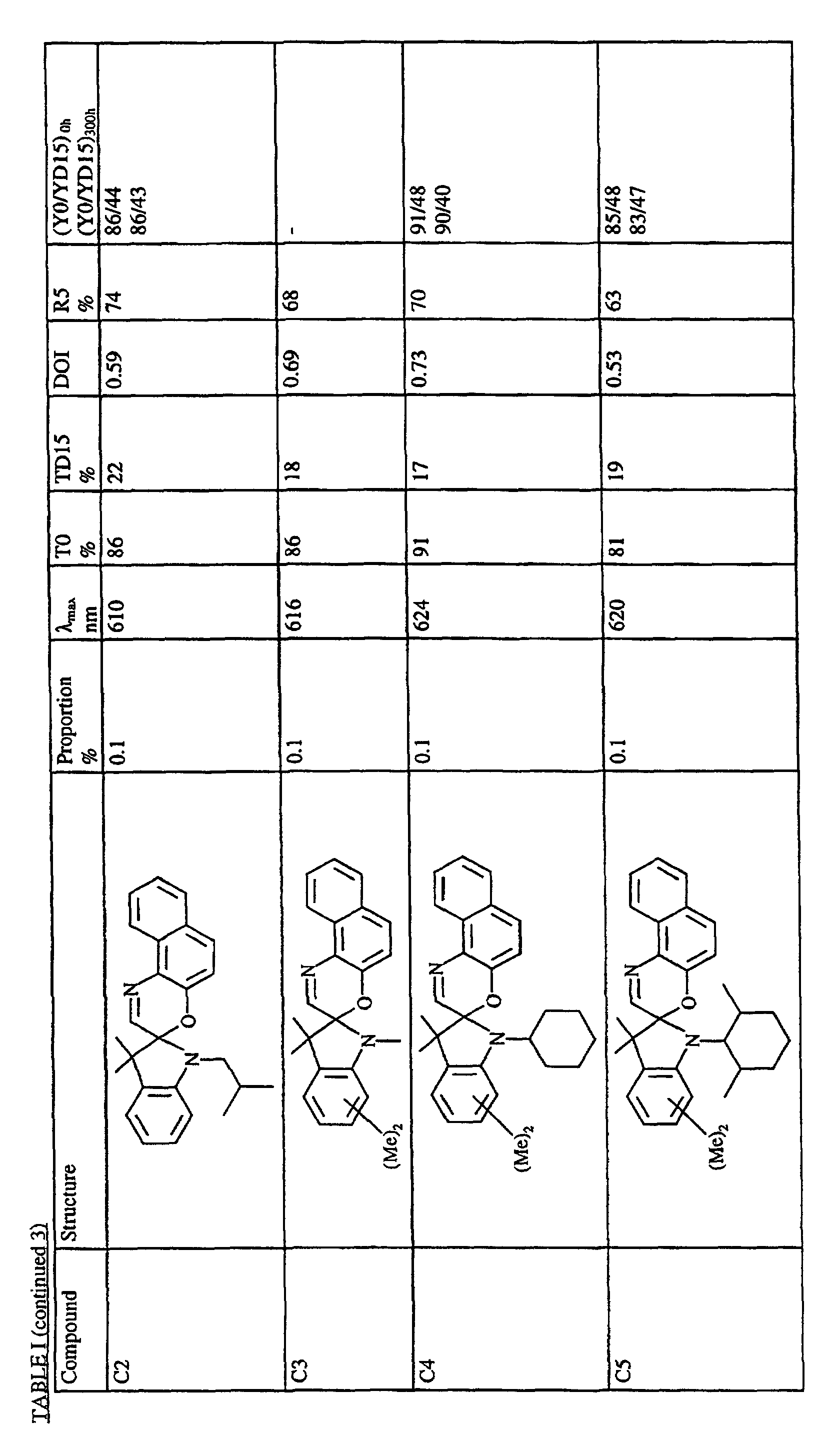

A comparison of the properties of Examples 1, 2 and 5 and of Comparative Examples C₁ and C₂, on the one hand, and Examples 3, 4 and 6 and Comparative Examples C₃-C₅, on the other hand, show that the compounds of the prior art with similar structure but without the polycyclic group according to the invention do not possess the advantageous combination of properties sought. In particular, it can be observed that the compounds of the invention have a better compromise between low initial coloration and strong induced optical density, and are photochemically very stable with little or no loss of colorability or decrease of the initial transmission.

## Claims

1. Photochromic compounds having the following general formula (I): 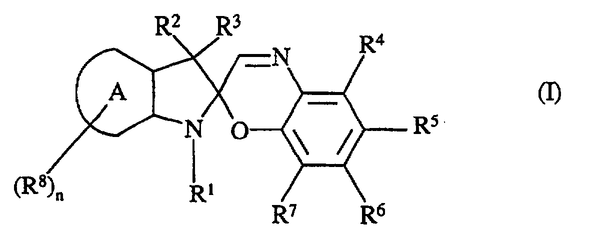 in which:
- R¹ is a polycyclic group formed by at least one alicyclic group linked to, bridged or condensed with at least one other aliphatic and/or aromatic ring, where the rings can optionally contain at least one heteroatom and at least one unsaturation,
- R², R³ are identical or different and they represent an alkyl group, straight or branched, of 1-12 carbon atoms, an alkenyl group, an alkynyl group, an aryl group, an alkylaryl group, a cycloalkyl group; or R² and R³ can, optionally, combine to form a carbocyclic or heterocyclic group having 5 to 10 atoms,
- R⁴, R⁵, R⁶ and R⁷ are identical or different and they represent:
■ hydrogen,
■ an alkyl, cycloalkyl, alkenyl, alkynyl, aryl, heteroaryl, aryloxy or aralkyl group, said group being optionally halogenated,
■ a halogen,
■ OR, SR, -OCOR, or -COOR, with R = H, alkyl and/or cycloalkyl and/or aryl,
■ a (poly)ether, a (poly)amide, a (poly)carbonate, a (poly)carbamate, a (poly)urea or a (poly)ester,
■ ∗ an amino radical which gives rise, once it is linked in (I), to a primary, secondary or tertiary amine, said amine being alkyl, aryl or aralkyl, mono- or disubstituted depending on its nature,
∗ or an aminocyclic radical containing, optionally, one or more heteroatoms,
■ or an electron withdrawing group selected, preferably, from the group comprising CF₃, CN, NO₂ and SCN,
- where at least two of the radicals R⁴, R⁵, R⁶, R⁷, can optionally carried form an assembly of at least one aromatic ring having 5 or 6 members or aliphatic ring having 5 to 7 members, said ring(s) comprising, optionally, at least one heteroatom, so as to form at least one heterocyclic ring, the latter being optionally substituted by one or more radicals, identical or different, and having the same definition as given above for R⁴ to R⁷,
- A represents a (hetero)aromatic ring (containing, for example, at least one nitrogen atom, which is possibly substituted by one or more radicals R⁸, identical or different, and having the same definition as given above for R⁴ to R⁷, except hydrogen,
- n is a whole number and when n ≥ 2, two of the radicals R⁸ can possibly combine to form at least one aromatic or heteroaromatic ring.

2. Compounds according to claim 1, **characterized in that** the polycyclic group R¹ is a bicyclic group.

3. Compounds according to claim 2, **characterized in that** the bicyclic group is asymmetric.

4. Compounds according to claim 1, **characterized in that** R¹ is selected from the following groups:

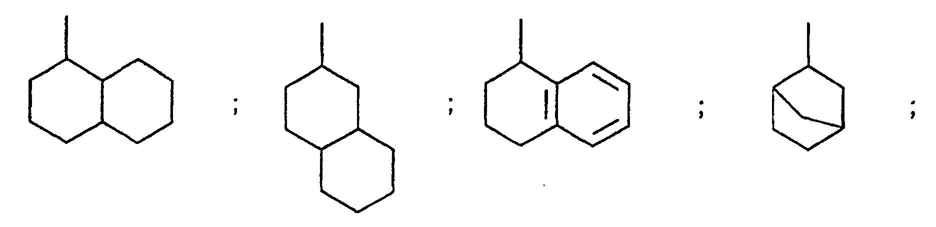

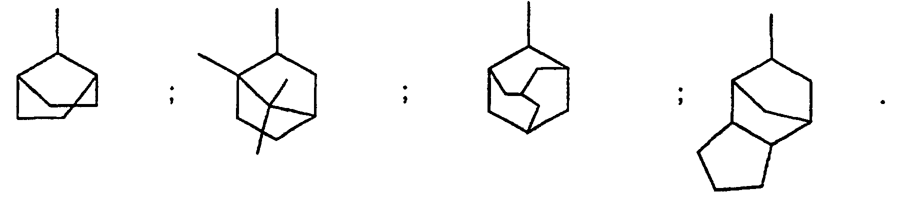

5. Compounds according to any one of claims I to 3, **characterized in that** the group R¹ is a substituted or unsubstituted norbornyl group.

6. Photochromic compounds according to any one of claims 1 to 5, **characterized in that** they have the following general formula: 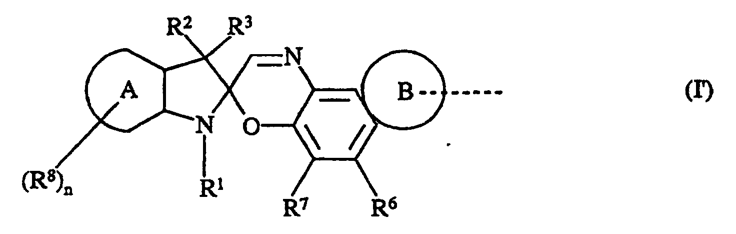 where A, R¹ to R³, R⁶ to R⁸ and n are as defined in the preceding claims, and B is an aromatic or aliphatic ring having 5 to 7 members, optionally comprising a heteroatom, which may or may not be substituted by one or more radicals, which may be identical or different, and have the same definition as given for R⁴ to R⁷ in the preceding claims, except hydrogen.

7. Photochromic compounds according to any one of claims 1 to 5, **characterized in that** they have the following general formula (I"): 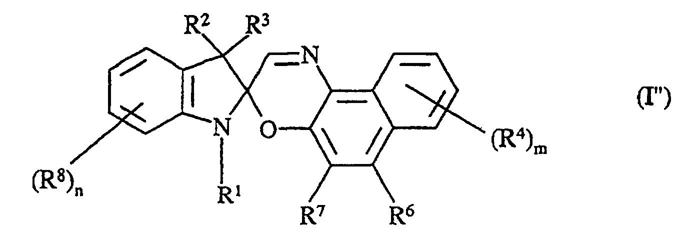 in which R¹-R⁴ and R⁶-R⁸ are defined as in claim 1, and n has values from 0 to 4, while m has values from 1 to 4.

8. Compounds according to any one of claims 1 to 7, **characterized in that** the groups R¹-R⁸ of formulas (I), (I') and (I") according to the invention, comprise and/or form at least one reactive polymerization and/or crosslinking group selected from the following: alkenyl, methacryloyl, acryloyl, acryloxyalkyl, methacryloxyalkyl and epoxy.

9. Compounds according to any one of claims 1 to 8, **characterized in that** they present the following formula: 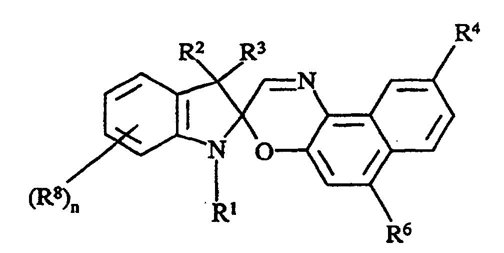 where
N-R¹ = 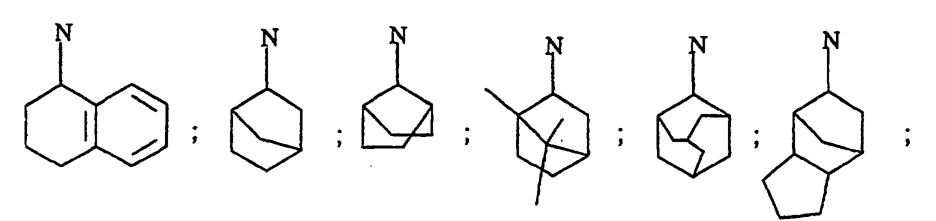
n = 0, 1 or 2,
R², R³ = C₁-C₅ alkyl,
R⁴ = H, OMe,
R⁶ = H, OMe or amino and
R⁸ = Me, OMe or CF₃.

10. (Co)polymer and/or crosslinkage, obtained by the polymerization and/or crosslinking of at least one monomer consisting of at least one photochromic compound according to claim 8.

11. (Co)polymer, **characterized in that** it is grafted by means of at least one of the photochromic compounds according to any one of claims 1 to 9.

12. Photochromic composition, **characterized in that** it comprises:
- at least one compound according to any one of claims 1 to 9 and/or at least one (co)polymer according to claim 10 or 11,
- and optionally at least one other photochromic compound and/or at least one dye and/or at least one stabilizer.

13. (Co)polymer matrix, **characterized in that** it comprises:
- at least one compound according to any one of claims 1 to 9,
- and/or at least one (co)polymer according to claim 10 or 11,
and/or at least one composition according to claim 12.

14. Matrix according to claim 13, **characterized in that** the (co)polymer is selected from the following list:
- alkyl, cycloalkyl, aryl or arylalkyl (mono, di, tri or tetra) polyacrylate or polymethacrylate optionally halogenated or comprising at least one ether and/or ester and/or carbonate and/or carbamate and/or thiocarbamate and/or urea and/or amide group,
- polystyrene, polycarbonate, polyepoxy, polyurethane, polythiourethane, polysiloxane, polyacrylonitrile, polyamide, aliphatic or aromatic polyester, polyvinyl acetate, cellulose acetate, cellulose triacetate, cellulose acetate-propionate or polyvinylbutyral,
- copolymers of two or more types of monomer or mixtures of monomers indicated above.

15. Ophthalmic or solar articles comprising:
- at least one compound according to any one of claims 1 to 9,
- and/or at least one (co)polymer according to claim 10 or 11,
- and/or at least one composition according to claim 11.

16. Article according to claim 15, **characterized in that** it consists of a lens.

17. Glass partitions and/or optical device comprising:
- at least one compound according to any one of claims 1 to 9,
- and/or at least one (co)polymer according to claim 10 or 11,
- and/or at least one composition according to claim 12,
- and/or at least one matrix according to claim 13 or 14.

## Patentansprüche

1. Photochrome Verbindungen der folgenden allgemeinen Formel (I) 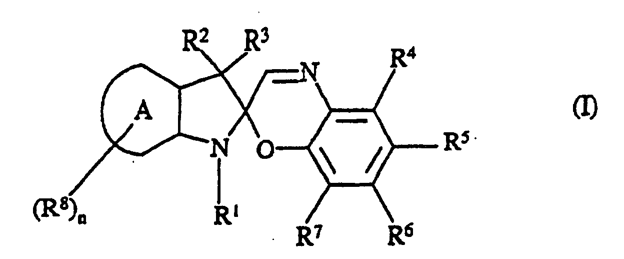 worin bedeuten:
- R¹ eine polycyclische Gruppe, die gebildet ist durch mindestens eine alicyclische Gruppe, die gebunden ist an, über eine Brücke gebunden ist an oder kondensiert ist mit mindestens einem anderen aliphatischen und/oder aromatischen Ring, wobei die Ringe gegebenenfalls mindestens ein Heteroatom und mindestens eine Unsättigung enthalten können,
- R², R³ gleich oder verschieden sind und eine unverzweigte oder verzweigte C₁-C₁₂-Alkylgruppe, -Alkenylgruppe, - Alkinylgruppe, -Arylgruppe, -Alkylarylgruppe, -Cycloalkylgruppe darstellen, oder R² und R³ gegebenenfalls miteinander kombiniert sein können unter Bildung einer carbocyclischen oder heterocyclischen Gruppe mit 5 bis 10 Atomen,
- R⁴, R⁵, R⁶ und R⁷ gleich oder verschieden sind und darstellen:
■ Wasserstoff,
■ eine Alkyl-, Cycloalkyl-, Alkenyl-, Alkinyl-, Aryl-, Heteroaryl-, Aryloxy- oder Aralkylgruppe, wobei die genannte Gruppe gegebenenfalls halogeniert ist,
■ ein Halogen,
■ OR, SR, -OCOR oder -COOR, worin R = H, Alkyl und/oder Cycloalkyl und/oder Aryl,
■ einen (Poly)Ether, ein (Poly)Amid, ein (Poly)Carbonat, ein (Poly)Carbamat, einen (Poly)Harnstoff oder einen (Poly)Ester,
■ ∗ einen Aminorest, der, wenn er einmal an (I) gebunden ist, ein primäres, sekundäres oder tertiäras Amin ergibt, wobei das genannte Amin je nach Art durch Alkyl, Aryl oder Aralkyl mono- oder disbstituiert ist,
∗ oder einem aminocyclischen Rest, der gegebenenfalls ein oder mehr Heteroatome enthält,
■ oder eine Elektronen anziehende Gruppe, vorzugsweise ausgewählt aus der Gruppe, die umfasst CF₃, CN, NO₂ und SCN,
- wobei mindestens zwei der Reste R⁴, R⁵, R⁶, R⁷ gegebenenfalls getragen werden können von einer Gruppe aus mindestens einem aromatischen Ring mit 5 oder 6 Gliedern oder einem aliphatischen Ring mit 5 bis 7 Gliedern, wobei der (die) genannte(n) Ring(e) gegebenenfalls mindestens ein Heteroatom umfasst (umfassen) unter Bildung mindestens eines heterocyclischen Ringes, der gegebenenfalls substituiert ist durch einen oder mehr gleiche oder verschiedene Reste, welche die gleiche Definition haben, wie sie oben für R⁴ bis R⁷ angegeben ist,
- A einen (hetero)aromatischen Ring (der beispielsweise mindestens ein Stickstoffatom enthält), der möglicherweise durch einen oder mehr gleiche oder verschiedene Reste R⁸ substituiert ist, welche die gleiche Definition haben, wie sie oben für R⁴ bis R⁷ angegeben ist, mit Ausnahme von Wasserstoff,
- n eine ganze Zahl, wobei dann, wenn n ≥ 2, zwei der Reste R⁸ möglicherweise miteinander verbunden sein können unter Bildung mindestens eines aromatischen oder heteroaromatischen Ringes.

2. Verbindungen nach Anspruch 1, **dadurch gekennzeichnet, dass** die polycyclische Gruppe R¹ eine bicyclische Gruppe ist.

3. Verbindungen nach Anspruch 2, **dadurch gekennzeichnet, dass** die bicyclische Gruppe asymmetrisch ist.

4. Verbindungen nach Anspruch 1, **dadurch gekennzeichnet, dass** R¹ ausgewählt ist aus den folgenden Gruppen:

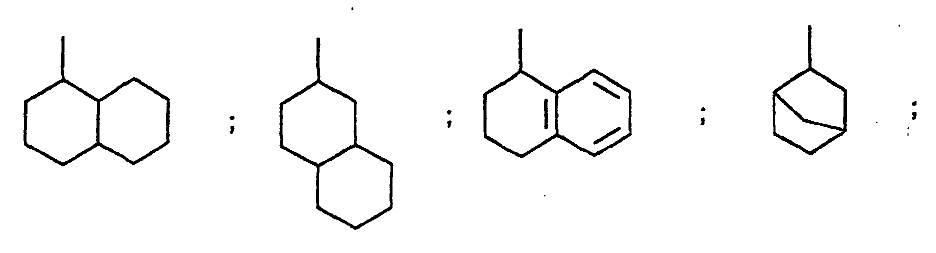

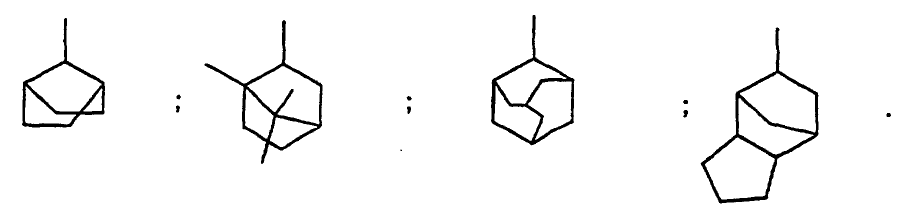

5. Verbindungen nach einem der Ansprüche 1 bis 3, **dadurch gekennzeichnet, dass** die Gruppe R¹ eine substituierte oder unsubstituierte Norbornylgruppe ist.

6. Photochrome Verbindungen nach einem der Ansprüche 1 bis 5, **dadurch gekennzeichnet, dass** sie die folgende allgemeine Formel haben: 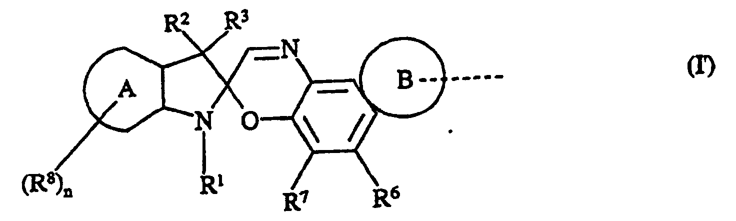 worin A, R¹ bis R³, R⁶ bis R⁸ und n wie in den vorhergehenden Ansprüchen definiert sind und B für einen aromatischen oder aliphatischen Ring mit 5 bis 7 Gliedern steht, der gegebenenfalls ein Heteroatom umfasst und der unsubstituiert sein kann oder substituiert sein kann durch einen oder mehr gleiche oder verschiedene Reste, welche die gleiche Definition haben, wie sie in den vorhergehenden Ansprüchen für R⁴ bis R⁷ angegeben ist, mit Ausnahme von Wasserstoff.

7. Photochrome Verbindungen nach einem der Ansprüche 1 bis 5, **dadurch gekennzeichnet, dass** sie die folgende allgemeine Formel (I") haben: 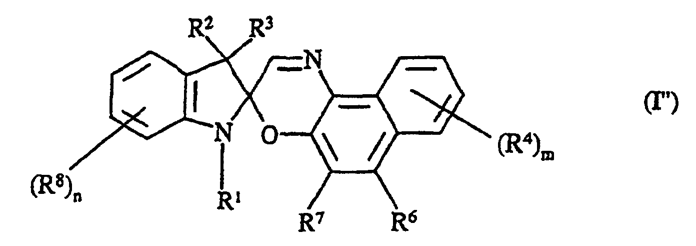 worin R¹ bis R⁴ und R⁶ bis R⁸ wie in Anspruch 1 definiert sind und n Werte von 0 bis 4 hat, während m Werte von 1 bis 4 hat.

8. Verbindungen nach einem der Ansprüche 1 bis 7, **dadurch gekennzeichnet, dass** die Gruppen R¹ bis R⁸ der erfindungsgemäßen Formeln (I), (I') und (I") umfassen und/oder bilden mindestens eine reaktionsfähige Polymerisationsgruppe und/oder vernetzende Gruppe, ausgewählt aus den folgenden Alkenyl, Methacryloyl, Acryloyl, Acryloxyalkyl, Methacryloxyalkyl und Epoxy.

9. Verbindungen nach einem der Ansprüche 1 bis 8, **dadurch gekennzeichnet, dass** sie die folgende Formel haben: 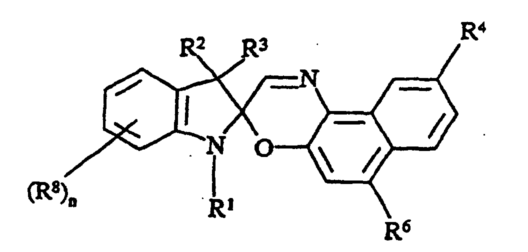 worin
N-R¹ = 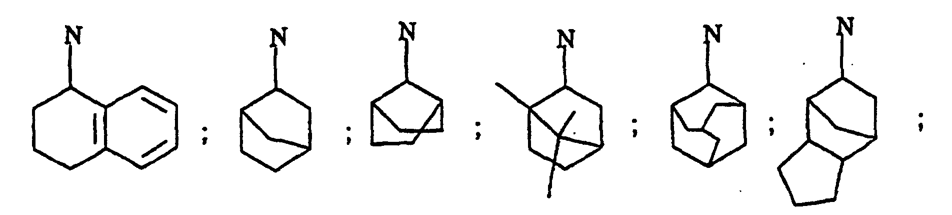
n = 0, 1 oder 2,
R², R³ = C₁-C₅-Alkyl,
R⁴ = H, OMe,
R⁶ = H, OMe oder Amino und
R⁸ = Me, OMe oder CF₃.

10. (Co)Polymer und/oder Vernetzungsprodukt, hergestellt durch Polymerisation und/oder Vernetzung mindestens eines Monomers, bestehend aus mindestens einer photochromen Verbindung nach Anspruch 8.

11. (Co)Polymer, **dadurch gekennzeichnet, dass** es bepfropft ist mit mindestens einer der photochromen Verbindungen nach einem der Ansprüche 1 bis 9.

12. Photochrome Zusammensetzung, **dadurch gekennzeichnet, dass** sie umfasst:
- mindestens eine Verbindung nach einem der Ansprüche 1 bis 9 und/oder mindestens ein (Co)Polymer nach Anspruch 10 oder 11, und
- gegebenenfalls mindestens eine andere (weitere) photochrome Verbindung und/oder mindestens einen Farbstoff und/oder mindestens einen Stabilisator.

13. (Co)Polymer-Matrix, **dadurch gekennzeichnet, dass** sie umfasst:
- mindestens eine Verbindung nach einem der Ansprüche 1 bis 9,
- und/oder mindestens ein (Co)Polymer nach Anspruch 10 oder 11,
- und/oder mindestens eine Zusammensetzung nach Anspruch 12.

14. Matrix nach Anspruch 13, **dadurch gekennzeichnet, dass** das (Co)Polymer ausgewählt ist aus der folgenden Liste:
- Alkyl-, Cycloalkyl-, Aryl- oder Arylalkyl (-mono-, -di-, -tri- oder -tetra)polyacrylat oder -polymethacrylat, das gegebenenfalls halogeniert ist oder mindestens eine Ether- und/oder Ester- und/oder Carbonat- und/oder Carbamat- und/oder Thiocarbamat- und/oder Harnstoff- und/oder Amidgruppe aufweist,
- Polystyrol, Polycarbonat, Polyepoxy, Polyurethan, Polythiourethan, Polysiloxan, Polyacrylnitril, Polyamid, aliphatischer oder aromatischer Polyester, Polyvinylacetat, Celluloseacetat, Cellulosetriacetat, Celluloseacetatpropionat oder Polyvinylbutyral,
- Copolymere von zwei oder mehr Typen von Monomeren oder einer Mischung von Monomeren, wie sie oben angegeben sind.

15. Ophthalmische oder Sonnenschutz- bzw. Solar-Artikel bzw. Gegenstände, die umfassen:
- mindestens eine Verbindung nach einem der Ansprüche 1 bis 9,
- und/oder mindestens ein (Co)Polymer nach Anspruch 10 oder 11,
- und/oder mindestens eine Zusammensetzung nach Anspruch 11.

16. Artikel bzw. Gegenstand nach Anspruch 15, **dadurch gekennzeichnet, dass** er aus einer Linse besteht.

17. Glastrennwände und/oder optische Einrichtung, die umfassen (umfasst):
- mindestens eine Verbindung nach einem der Ansprüche 1 bis 9,
- und/oder mindestens ein (Co)Polymer nach Anspruch 10 oder 11,
- und/oder mindestens eine Zusammensetzung nach Anspruch 12,
- und/oder mindestens eine Matrix nach Anspruch 13 oder 14.

## Revendications

1. Composés photochromiques ayant la formule générale (I) suivante : 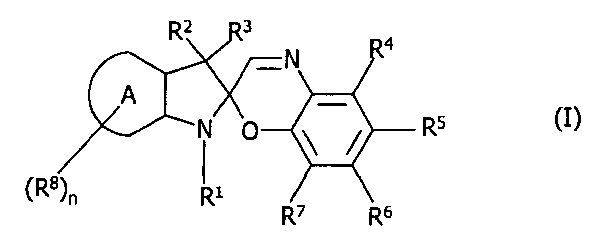 dans laquelle,
- R¹ est un groupement polycyclique formé par au moins un groupement alicyclique relié à, ponté ou condensé avec au moins un autre cycle aliphatique et/ou aromatique, ces cycles pouvant, éventuellement, contenir au moins un hétéroatome et au moins une insaturation,
- R², R³ sont identiques ou différents et représentent un groupement alkyle, linéaire ou ramifié, de 1 à 12 atomes de carbone, alcényle, alcynyle, aryle, alkylaryle, cycloalkyle, ou bien R² et R³ peuvent, éventuellement, être pris ensemble pour former un groupe carbocyclique ou hétérocyclique de 5 à 10 atomes.
- R⁴, R⁵, R⁶ et R⁷ sont identiques ou différents et représentent :
• l'hydrogène
• un groupement alkyle, cycloalkyle, alcényle, alcynyle, aryle, hétéroaryle, aryloxy ou aralkyle, ledit groupement étant éventuellement halogéné,
• un halogène,
• OR, SR, -OCOR ou -COOR, avec R = H, alkyle et/ou cycloalkyle et/ou aryle,
• un (poly)éther, un (poly)amide, un (poly)carbonate, un (poly)carbamate, une (poly)urée ou un (poly)ester,
• ∗ un radical amino donnant naissance, une fois lié dans (I), à une amine primaire, secondaire ou tertiaire, ladite amine étant alkyl-, aryl- ou aralkyl-, mono- ou disubstituée selon sa nature,
∗ ou un radical aminocyclique contenant, éventuellement, un ou plusieurs hétéroatomes,
• ou un groupement électroattracteur choisi de préférence dans le groupe comprenant CF₃, CN, NO₂ et SCN,
- au moins deux des radicaux R⁴, R⁵, R⁶ et R⁷ pouvant éventuellement former ensemble au moins un cycle aromatique ayant 5
ou 6 chaînons ou aliphatique ayant 5 à 7 chaînons, ledit ou lesdits cycle(s) comprenant, éventuellement, au moins un hétéroatome, de façon à former au moins un noyau hétérocyclique, ce dernier étant éventuellement substitué par un ou plusieurs radicaux, identiques ou différents, et répondant à la même définition que celle donnée supra pour R⁴ à R⁷,
- A représente un cycle (hétéro)aromatique (contenant par exemple au moins un atome d'azote) qui est éventuellement substitué par un ou plusieurs radicaux R⁸, identiques ou différents, et répondant à la même définition que celle donnée supra pour R⁴ à R⁷, sauf l'hydrogène,
- n est un nombre entier et quand n ≥ 2, deux des radicaux R⁸ peuvent éventuellement être pris ensemble pour former au moins un cycle aromatique ou hétéroatomatique.

2. Composés selon la revendication 1, **caractérisés en ce que** le groupement polycyclique R¹ est un groupe bicyclique.

3. Composés selon la revendication 2, **caractérisés en ce que** le groupe bicyclique est dissymétrique.

4. Composés selon la revendication 1, **caractérisés en ce que** R¹ est choisi parmi les groupements suivants :

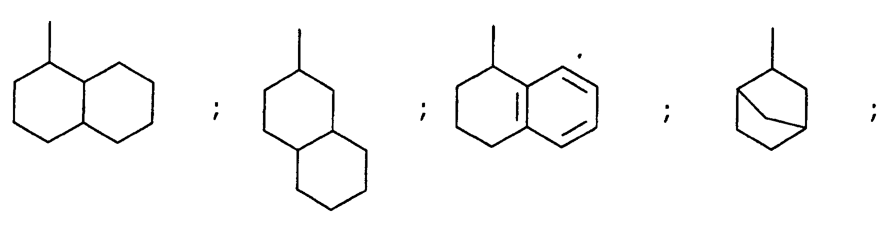

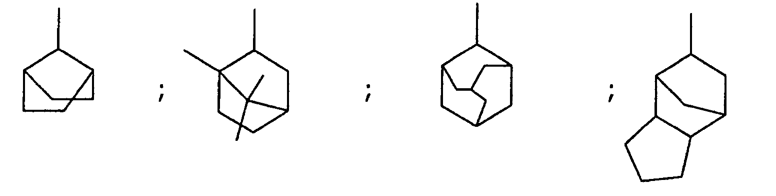

5. Composés selon l'une quelconque des revendications 1 à 3, **caractérisés en ce que** le groupement R¹ est un groupement norbornyle, substitué ou non.

6. Composés photochromiques selon l'une quelconque des revendications 1 à 5, **caractérisés en ce qu'**ils répondent à la formule générale suivante : 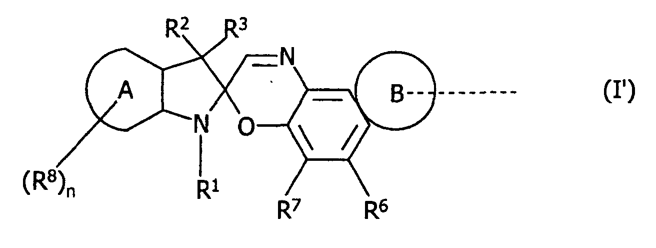 où A, R¹ à R³, R⁶ à R⁸ et n sont tels que définis dans les revendications précédentes, et B est un cycle aromatique ou aliphatique de 5 à 7 chaînons, comprenant éventuellement un hétéroatome, qui peut être substitué ou non par un ou plusieurs radicaux, qui peuvent être identiques ou différents, et répondre à la même définition que celle donnée pour R⁴ à R⁷ dans les revendications précédentes, sauf l'hydrogène.

7. Composés photochromiques selon l'une quelconque des revendications 1 à 5, **caractérisés en ce qu'**ils ont la formule générale (I") suivante : 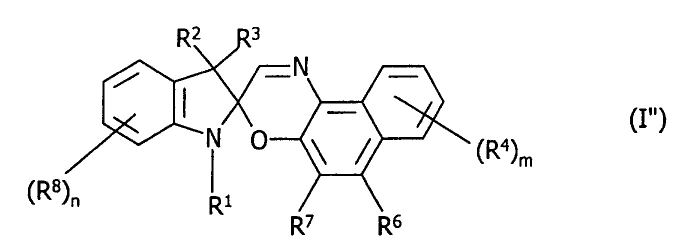 dans laquelle R¹ à R⁴ et R⁶ à R⁸ sont tels que définis dans la revendication 1, et n prend des valeurs de 0 à 4 tandis que m prend des valeurs de 1 à 4.

8. Composés selon l'une quelconque des revendications 1 à 7, **caractérisés en ce que** les groupements R¹ à R⁸ des formules (I), (I') et (I") selon l'invention, comprennent et/ou forment au moins un groupe réactif de polymérisation et/ou réticulation, choisi parmi les suivants : alcényle, méthacryloyle, acryloyle, acryloxyalkyle, méthacryloxylalkyle et époxy.

9. Composés selon l'une quelconque des revendications 1 à 8, **caractérisés en ce qu'**ils présentent la formule suivante : 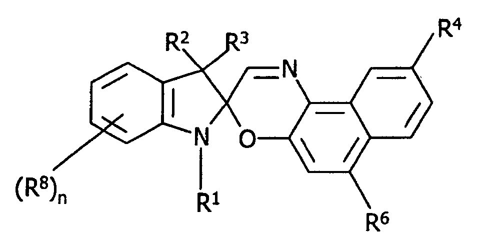 où
N-R¹ = 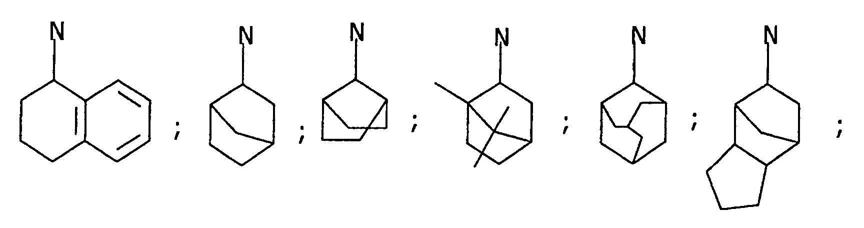
n = 0, 1 ou 2,
R², R³ = alkyle en C₁-C₅,
R⁴ = H, OMe,
R⁶ = H, OMe ou amino, et
R⁸ = Me, OMe ou CF₃.

10. (Co)polymère et/ou réticulat obtenu par polymérisation et/ou réticulation d'au moins un monomère constitué par au moins un composé photochromique selon la revendication 8.

11. (Co)polymère, **caractérisé en ce qu'**il est greffé à l'aide d'au moins l'un des composés photochromiques selon l'une quelconque des revendications 1 à 9.

12. Composition photochromique, **caractérisée en ce qu'**elle comprend :
- au moins un composé selon l'une quelconque des revendications 1 à 9 et/ou au moins un (co)polymère selon la revendication 10 ou 11,
- et éventuellement au moins un autre composé photochromique et/ou au moins un colorant et/ou au moins un stabilisant.

13. Matrice (co)polymère, **caractérisée en ce qu'**elle comprend :
- au moins un composé selon l'une quelconque des revendications 1 à 9,
- et/ou au moins un (co)polymère selon la revendication 10 ou 11,
- et/ou au moins une composition selon la revendication 12.

14. Matrice selon la revendication 13, **caractérisée en ce que** le (co)polymère est choisi dans la liste suivante :
- polyacrylate ou polyméthacrylate d'alkyle, de cycloalkyle, d'aryle ou d'arylalkyle (mono, di, tri ou tétra) éventuellement halogéné ou comportant au moins un groupement éther et/ou ester et/ou carbonate et/ou carbamate et/ou thiocarbamate et/ou urée et/ou amide,
- polystyrène, polycarbonate, polyépoxy, polyuréthane, polythiouréthane, polysiloxane, polyacrylonitrile, polyamide, polyester aliphatique ou aromatique, acétate de polyvinyle, acétate de cellulose, triacétate de cellulose, acétate-propionate de cellulose ou polyvinylbutyral,
- copolymères de deux ou plusieurs types de monomères ou mélanges de polymères visés supra.

15. Articles ophtalmiques ou solaires comprenant :
- au moins un composé selon l'une quelconque des revendications 1 à 9,
- et/ou au moins un (co)polymère selon la revendication 10 ou 11,
- et/ou au moins une composition selon la revendication 11.

16. Article selon la revendication 15, **caractérisé en ce qu'**il est constitué par une lentille.

17. Vitrage et/ou dispositif optique comprenant :
- au moins un composé selon l'une quelconque des revendications 1 à 9,
- et/ou au moins un (co)polymère selon la revendication 10 ou 11,
- et/ou au moins une composition selon la revendication 12,
- et/ou au moins une matrice selon la revendication 13 ou 14.
